# EUROPEAN PATENT SPECIFICATION

(11) **EP 1 987 838 B1**
(45) Date of publication and mention of the grant of the patent: **13.01.2016**
(21) Application number: 07702135.0
(22) Date of filing: 19.01.2007
(51) Int. Cl.: A61K 38/50, C12N 9/78, C12N 9/96, C12N 11/08, A61P 35/00

(54) **NOVEL COMPOUND FOR TREATMENT OF TUMOR**
NEUE VERBINDUNG ZUR TUMORTHERAPIE
NOUVEAU COMPOSÉ DE TRAITEMENT DE TUMEUR

(30) Priority: 20.01.2006 CN 200610011246
(43) Date of publication of application: 05.11.2008
(73) Proprietor: Tsinghua University, Haidian District, Beijing 100084 (CN); Protgen Ltd., 100085 Beijing (CN)
(72) Inventor: LUO, Yongzhang, Beijing 100084 (CN); ZHOU, Hao, Beijing 100084 (CN); LEI, Qingxin, Beijing 100085 (CN); CHANG, Guodong, Beijing 100085 (CN)
(74) Representative: Nederlandsch Octrooibureau
(86) International application number: PCT/CN2007/000203
(87) International publication number: WO 2007/082482

(56) References cited:
- WO-A1-90/15628
- WO-A1-2004/001048
- WO-A2-01/83774
- WO-A2-2004/046309
- CN-A- 1 255 944
- CN-A- 1 518 595
- KOCHENDOERFER G: "CHEMICAL AND BIOLOGICAL PROPERTIES OF POLYMER-MODIFIED PROTEINS" EXPERT OPINION ON BIOLOGICAL THERAPY, vol. 3, no. 8, 2003, pages 1253-1261, XP008036528
- HOLTSBERG F W ET AL: "Poly(ethylene glycol) (PEG) conjugated arginine deiminase: effects of PEG formulations on its pharmacological properties" JOURNAL OF CONTROLLED RELEASE, vol. 80, no. 1-3, 2002, pages 259-271, XP004348640
- GAERTNER H F ET AL: "SITE-SPECIFIC ATTACHMENT OF FUNCTIONALIZED POLY(ETHYLENE GLYCOL) TOTHE AMINO TERMINUS OF PROTEINS" BIOCONJUGATE CHEMISTRY, vol. 7, no. 1, 1996, pages 38-44, XP002944821
- EDWARDS C K ET AL: "Design of PEGylated Soluble Tumor Necrosis Factor Receptor Type I (PEG sTNF-RI) for Chronic Inflammatory Diseases" ADVANCED DRUG DELIVERY REVIEWS, vol. 55, 2003, pages 1315-1336, XP003008526
- WAN ET AL: "An enzyme-linked immunosorbent assay for host cell protein contaminants in recombinant PEGylated staphylokinase mutant SY161" JOURNAL OF PHARMACEUTICAL AND BIOMEDICAL ANALYSIS, vol. 28, 2002, pages 953-963, XP002987234
- DATABASE BIOSIS [Online] BIOSCIENCES INFORMATION SERVICE, PHILADELPHIA, PA, US; 2002, LEE H ET AL: "Preparation and characterization of mono-PEGylated epidermal growth factor: Evaluation of in vitro biologic activity" XP002562904 Database accession no. PREV200200438985 & LEE H ET AL: PHARMACEUTICAL RESEARCH, vol. 19, no. 6, 2002, pages 845-851,
- ENSOR C.M. ET AL.: 'Pegylated Arginine Deiminase (ADI-SS PEG20,000 mw) Inhibits Human Melanomas and Hepatocellular Carcinomas in Vitro and in Vivo' CANCER RESEARCH vol. 62, 01 October 2002, pages 5443 - 5450, XP002230768
- JIANG Z. ET AL.: 'Chemistry for Pegylation of Protein and Peptide Molecules' CHINESE JOURNAL OF ORGANIC CHEMISTRY vol. 23, no. 12, 2003, pages 1340 - 1347, XP008129028

### SEQUENCE LISTING

<110> Tsinghua University PROTGEN LTD.
<120> A novel conjugate for the treatment of tumors
<130> P2007434C
<150> 200610011246.4
   <151> 2006-01-20
<160> 5
<170> PatentIn version 3.3
<210> 1
   <211> 409
   <212> PRT
   <213> Mycoplasma hominis
<400> 1
   Met Ser Val Phe Asp Ser Lys Phe Asn Gly Ile His Val Tyr Ser Glu
   1               5                   10                  15
   Ile Gly Glu Leu Glu Thr Val Leu Val His Glu Pro Gly Arg Glu Ile
               20                  25                  30
   Asp Tyr Ile Thr Pro Ala Arg Leu Asp Glu Leu Leu Phe Ser Ala Ile
           35                  40                  45
   Leu Glu Ser His Asp Ala Arg Lys Glu His Gln Ser Phe Val Lys Ile
       50                  55                  60
   Met Lys Asp Arg Gly Ile Asn Val Val Glu Leu Thr Asp Leu Val Ala
   65                  70                  75                  80
   Glu Thr Tyr Asp Leu Ala Ser Lys Ala Ala Lys Glu Glu Phe Ile Glu
                   85                  90                  95
   Thr Phe Leu Glu Glu Thr Val Pro Val Leu Thr Glu Ala Asn Lys Lys
               100                 105                 110
   Ala Val Arg Ala Phe Leu Leu Ser Lys Pro Thr His Glu Met Val Glu
           115                 120                 125
   Phe Met Met Ser Gly Ile Thr Lys Tyr Glu Leu Gly Val Glu Ser Glu
       130                 135                 140
   Asn Glu Leu Ile Val Asp Pro Met Pro Asn Leu Tyr Phe Thr Arg Asp
   145                 150                 155                 160
   Pro Phe Ala Ser Val Gly Asn Gly Val Thr Ile His Phe Met Arg Tyr
                   165                 170                 175
   Ile Val Arg Arg Arg Glu Thr Leu Phe Ala Arg Phe Val Phe Arg Asn
               180                 185                 190
   His Pro Lys Leu Val Lys Thr Pro Trp Tyr Tyr Asp Pro Ala Met Lys
           195                 200                 205
   Met Pro Ile Glu Gly Gly Asp Val Phe Ile Tyr Asn Asn Glu Thr Leu
       210                 215                 220
   Val Val Gly Val Ser Glu Arg Thr Asp Leu Asp Thr Ile Thr Leu Leu
   225                 230                 235                 240
   Ala Lys Asn Ile Lys Ala Asn Lys Glu Val Glu Phe Lys Arg Ile Val
                   245                 250                 255
   Ala Ile Asn Val Pro Lys Trp Thr Asn Leu Met His Leu Asp Thr Trp
               260                 265                 270
   Leu Thr Met Leu Asp Lys Asn Lys Phe Leu Tyr Ser Pro Ile Ala Asn
           275                 280                 285
   Asp Val Phe Lys Phe Trp Asp Tyr Asp Leu Val Asn Gly Gly Ala Glu
       290                 295                 300
   Pro Gln Pro Gln Leu Asn Gly Leu Pro Leu Asp Lys Leu Leu Ala Ser
   305                 310                 315                 320
   Ile Ile Asn Lys Glu Pro Val Leu Ile Pro Ile Gly Gly Ala Gly Ala
                   325                 330                 335
   Thr Glu Met Glu Ile Ala Arg Glu Thr Asn Phe Asp Gly Thr Asn Tyr
               340                 345                 350
   Leu Ala Ile Lys Pro Gly Leu Val Ile Gly Tyr Asp Arg Asn Glu Lys
           355                 360                 365
   Thr Asn Ala Ala Leu Lys Ala Ala Gly Ile Thr Val Leu Pro Phe His
       370                 375                 380
   Gly Asn Gln Leu Ser Leu Gly Met Gly Asn Ala Arg Cys Met Ser Met
   385                 390                 395                 400
   Pro Leu Ser Arg Lys Asp Val Lys Trp
                   405
<210> 2
   <211> 408
   <212> PRT
   <213> Mycoplasma hominis
<400> 2
   Ser Val Phe Asp Ser Lys Phe Asn Gly Ile His Val Tyr Ser Glu Ile
   1               5                   10                  15
   Gly Glu Leu Glu Thr Val Leu Val His Glu Pro Gly Arg Glu Ile Asp
               20                  25                  30
   Tyr Ile Thr Pro Ala Arg Leu Asp Glu Leu Leu Phe Ser Ala Ile Leu
           35                  40                  45
   Glu Ser His Asp Ala Arg Lys Glu His Gln Ser Phe Val Lys Ile Met
       50                  55                  60
   Lys Asp Arg Gly Ile Asn Val Val Glu Leu Thr Asp Leu Val Ala Glu
   65                  70                  75                  80
   Thr Tyr Asp Leu Ala Ser Lys Ala Ala Lys Glu Glu Phe Ile Glu Thr
                   85                  90                  95
   Phe Leu Glu Glu Thr Val Pro Val Leu Thr Glu Ala Asn Lys Lys Ala
               100                 105                 110
   Val Arg Ala Phe Leu Leu Ser Lys Pro Thr His Glu Met Val Glu Phe
           115                 120                 125
   Met Met Ser Gly Ile Thr Lys Tyr Glu Leu Gly Val Glu Ser Glu Asn
       130                 135                 140
   Glu Leu Ile Val Asp Pro Met Pro Asn Leu Tyr Phe Thr Arg Asp Pro
   145                 150                 155                 160
   Phe Ala Ser Val Gly Asn Gly Val Thr Ile His Phe Met Arg Tyr Ile
                   165                 170                 175
   Val Arg Arg Arg Glu Thr Leu Phe Ala Arg Phe Val Phe Arg Asn His
               180                 185                 190
   Pro Lys Leu Val Lys Thr Pro Trp Tyr Tyr Asp Pro Ala Met Lys Met
           195                 200                 205
   Pro Ile Glu Gly Gly Asp Val Phe Ile Tyr Asn Asn Glu Thr Leu Val
       210                 215                 220
   Val Gly Val Ser Glu Arg Thr Asp Leu Asp Thr Ile Thr Leu Leu Ala
   225                 230                 235                 240
   Lys Asn Ile Lys Ala Asn Lys Glu Val Glu Phe Lys Arg Ile Val Ala
                   245                 250                 255
   Ile Asn Val Pro Lys Trp Thr Asn Leu Met His Leu Asp Thr Trp Leu
               260                 265                 270
   Thr Met Leu Asp Lys Asn Lys Phe Leu Tyr Ser Pro Ile Ala Asn Asp
           275                 280                 285
   Val Phe Lys Phe Trp Asp Tyr Asp Leu Val Asn Gly Gly Ala Glu Pro
       290                 295                 300
   Gln Pro Gln Leu Asn Gly Leu Pro Leu Asp Lys Leu Leu Ala Ser Ile
   305                 310                 315                 320
   Ile Asn Lys Glu Pro Val Leu Ile Pro Ile Gly Gly Ala Gly Ala Thr
                   325                 330                 335
   Glu Met Glu Ile Ala Arg Glu Thr Asn Phe Asp Gly Thr Asn Tyr Leu
               340                 345                 350
   Ala Ile Lys Pro Gly Leu Val Ile Gly Tyr Asp Arg Asn Glu Lys Thr
           355                 360                 365
   Asn Ala Ala Leu Lys Ala Ala Gly Ile Thr Val Leu Pro Phe His Gly
       370                 375                 380
   Asn Gln Leu Ser Leu Gly Met Gly Asn Ala Arg Cys Met Ser Met Pro
   385                 390                 395                 400
   Leu Ser Arg Lys Asp Val Lys Trp
                   405
<210> 3
   <211> 417
   <212> PRT
   <213> Mycoplasma hominis
<400> 3
   Met Gly Gly Ser His His His His His Ser Val Phe Asp Ser Lys Phe
   1               5                   10                  15
   Asn Gly Ile His Val Tyr Ser Glu Ile Gly Glu Leu Glu Thr Val Leu
               20                  25                  30
   Val His Glu Pro Gly Arg Glu Ile Asp Tyr Ile Thr Pro Ala Arg Leu
           35                  40                  45
   Asp Glu Leu Leu Phe Ser Ala Ile Leu Glu Ser His Asp Ala Arg Lys
       50                  55                  60
   Glu His Gln Ser Phe Val Lys Ile Met Lys Asp Arg Gly Ile Asn Val
   65 70 75 80
   Val Glu Leu Thr Asp Leu Val Ala Glu Thr Tyr Asp Leu Ala Ser Lys
   85 90 95
   Ala Ala Lys Glu Glu Phe Ile Glu Thr Phe Leu Glu Glu Thr Val Pro
   100 105 110
   Val Leu Thr Glu Ala Asn Lys Lys Ala Val Arg Ala Phe Leu Leu Ser
   115 120 125
   Lys Pro Thr His Glu Met Val Glu Phe Met Met Ser Gly Ile Thr Lys
   130 135 140
   Tyr Glu Leu Gly Val Glu Ser Glu Asn Glu Leu Ile Val Asp Pro Met
   145 150 155 160
   Pro Asn Leu Tyr Phe Thr Arg Asp Pro Phe Ala Ser Val Gly Asn Gly
   165 170 175
   Val Thr Ile His Phe Met Arg Tyr Ile Val Arg Arg Arg Glu Thr Leu
   180 185 190
   Phe Ala Arg Phe Val Phe Arg Asn His Pro Lys Leu Val Lys Thr Pro
   195 200 205
   Trp Tyr Tyr Asp Pro Ala Met Lys Met Pro Ile Glu Gly Gly Asp Val
   210 215 220
   Phe Ile Tyr Asn Asn Glu Thr Leu Val Val Gly Val Ser Glu Arg Thr
   225 230 235 240
   Asp Leu Asp Thr Ile Thr Leu Leu Ala Lys Asn Ile Lys Ala Asn Lys
   245 250 255
   Glu Val Glu Phe Lys Arg Ile Val Ala Ile Asn Val Pro Lys Trp Thr
   260 265 270
   Asn Leu Met His Leu Asp Thr Trp Leu Thr Met Leu Asp Lys Asn Lys
   275 280 285
   Phe Leu Tyr Ser Pro Ile Ala Asn Asp Val Phe Lys Phe Trp Asp Tyr
   290 295 300
   Asp Leu Val Asn Gly Gly Ala Glu Pro Gln Pro Gln Leu Asn Gly Leu
   305 310 315 320
   Pro Leu Asp Lys Leu Leu Ala Ser Ile Ile Asn Lys Glu Pro Val Leu
   325 330 335
   Ile Pro Ile Gly Gly Ala Gly Ala Thr Glu Met Glu Ile Ala Arg Glu
   340 345 350
   Thr Asn Phe Asp Gly Thr Asn Tyr Leu Ala Ile Lys Pro Gly Leu Val
   355 360 365
   Ile Gly Tyr Asp Arg Asn Glu Lys Thr Asn Ala Ala Leu Lys Ala Ala
   370 375 380
   Gly Ile Thr Val Leu Pro Phe His Gly Asn Gln Leu Ser Leu Gly Met
   385 390 395 400
   Gly Asn Ala Arg Cys Met Ser Met Pro Leu Ser Arg Lys Asp Val Lys
   405 410 415
   Trp
<210> 4
   <211> 416
   <212> PRT
   <213> Mycoplasma hominis
<400> 4
   Gly Gly Ser His His His His His Ser Val Phe Asp Ser Lys Phe Asn
   1 5 10 15
   Gly Ile His Val Tyr Ser Glu Ile Gly Glu Leu Glu Thr Val Leu Val
   20 25 30
   His Glu Pro Gly Arg Glu Ile Asp Tyr Ile Thr Pro Ala Arg Leu Asp
   35 40 45
   Glu Leu Leu Phe Ser Ala Ile Leu Glu Ser His Asp Ala Arg Lys Glu
   50 55 60
   His Gln Ser Phe Val Lys Ile Met Lys Asp Arg Gly Ile Asn Val Val
   65 70 75 80
   Glu Leu Thr Asp Leu Val Ala Glu Thr Tyr Asp Leu Ala Ser Lys Ala
   85 90 95
   Ala Lys Glu Glu Phe Ile Glu Thr Phe Leu Glu Glu Thr Val Pro Val
   100 105 110
   Leu Thr Glu Ala Asn Lys Lys Ala Val Arg Ala Phe Leu Leu Ser Lys
   115 120 125
   Pro Thr His Glu Met Val Glu Phe Met Met Ser Gly Ile Thr Lys Tyr
       130                 135                 140
   Glu Leu Gly Val Glu Ser Glu Asn Glu Leu Ile Val Asp Pro Met Pro
   145                 150                 155                 160
   Asn Leu Tyr Phe Thr Arg Asp Pro Phe Ala Ser Val Gly Asn Gly Val
                   165                 170                 175
   Thr Ile His Phe Met Arg Tyr Ile Val Arg Arg Arg Glu Thr Leu Phe
               180                 185                 190
   Ala Arg Phe Val Phe Arg Asn His Pro Lys Leu Val Lys Thr Pro Trp
           195                 200                 205
   Tyr Tyr Asp Pro Ala Met Lys Met Pro Ile Glu Gly Gly Asp Val Phe
       210                 215                 220
   Ile Tyr Asn Asn Glu Thr Leu Val Val Gly Val Ser Glu Arg Thr Asp
   225                 230                 235                 240
   Leu Asp Thr Ile Thr Leu Leu Ala Lys Asn Ile Lys Ala Asn Lys Glu
                   245                 250                 255
   Val Glu Phe Lys Arg Ile Val Ala Ile Asn Val Pro Lys Trp Thr Asn
               260                 265                 270
   Leu Met His Leu Asp Thr Trp Leu Thr Met Leu Asp Lys Asn Lys Phe
           275                 280                 285
   Leu Tyr Ser Pro Ile Ala Asn Asp Val Phe Lys Phe Trp Asp Tyr Asp
       290                 295                 300
   Leu Val Asn Gly Gly Ala Glu Pro Gln Pro Gln Leu Asn Gly Leu Pro
   305                 310                 315                 320
   Leu Asp Lys Leu Leu Ala Ser Ile Ile Asn Lys Glu Pro Val Leu Ile
                   325                 330                 335
   Pro Ile Gly Gly Ala Gly Ala Thr Glu Met Glu Ile Ala Arg Glu Thr
               340                 345                 350
   Asn Phe Asp Gly Thr Asn Tyr Leu Ala Ile Lys Pro Gly Leu Val Ile
           355                 360                 365
   Gly Tyr Asp Arg Asn Glu Lys Thr Asn Ala Ala Leu Lys Ala Ala Gly
       370                 375                 380
   Ile Thr Val Leu Pro Phe His Gly Asn Gln Leu Ser Leu Gly Met Gly
   385                 390                 395                 400
   Asn Ala Arg Cys Met Ser Met Pro Leu Ser Arg Lys Asp Val Lys Trp
                   405                 410                 415
<210> 5
   <211> 9
   <212> PRT
   <213> Artificial
<220>
   <223> His-tag
<400> 5
   Met Gly Gly Ser His His His His His
   1               5

## Description

### Field of the invention

The present invention relates to methods for preparing recombinant arginine deiminase with bioactivity and metabolic stability. The present invention also provides pharmaceutical conjugates comprising the arginine deiminase and pharmaceutical compositions comprising the arginine deiminase. The present invention further provides the arginine deiminase and the pharmaceutical composition for preventing, diagnosing, and treating tumors.

### Background of the invention

Hepatoma and malignant melanoma are diseases which are fatal to most of the patients within one year after diagnosis. Hepatoma is one of the most common malignant tumors in China, which develops fast and is hard to cure. The therapeutic effect is not satisfying and the patients suffering from hepatoma typically has a short life span.That is why hepatoma is called "the king of the cancers". The data of current investigation show that the annual mortality of hepatoma in China is 20.4 persons/ 100,000 persons, which is 18.8% of all the malignant tumors. The mortality rose from the third place in 1970s to the second place, which is second only to lung cancer in the city and to stomach cancer in the countryside. The malignant melanoma is an acute and malignant cancer, the incidence of which increases fastest in America. Meanwhile the dermal melanocytoma is 5% of all the dermal malignant cancers, while more than 75% of the patients who die of malignant cancers are those suffering from malignant melanoma. In 2003, about 60,000 Americans have been diagnosed to be suffering from melanoma, wherein 10,000 cases are lethal. If melanoma can not be treated in the early stage, it can develop into malignant tumor and transfer to the whole body with a great efficiency, unlike the other tumors.

The methods for specifically eliminating some essential amino acids from the blood can be used for treating certain cancers. In the methods, a famous example is the treatment of acute lymphocytic leukemia by using L-asparaginase to reduce the concentration of the asparagine in blood. The commonly-used L-asparaginase is isolated from *Escherichia coli.* But due to the inherent antigenicity and the short circulating half life, the application of the enzyme is limited greatly (Y. K. Park et al., Anticancer Res., 1: 373-376 (1981)). The half life can be prolonged remarkedly and the antigenicity can be reduced significantly, if L-asparaginase of the *Escherichia coli* is modified by polyethylene glycol (Y. K. Park et al., Anticancer Res., 1: 373-376 (1981), Y. Kamisald et al., J Pharmacol. Exp. Ther., 216: 410-414(1981), Y. Kamisaki, Gann., 73: 470-474(1982)). Although it is possible to treat some tumors to some extent by eliminating certain essential amino acids, the essential amino acids are also required by the growth of the normal cells. As a result, the reduction of certain essential amino acids in blood leads to some severe side effects.

It has been shown that some tumor cells have metabolic mode which is different from normal cells. The demand for certain types of amino acids by the tumor cells is also different from the normal cells. Based on the theory, it is found that some tumors, e.g., hepatoma and malignant melanoma, can be controlled with little effect on normal cells, by degrading certian non-essential amino acids, e.g. arginine. One arginine deiminase from *Pseudomonas pudita* can inhibit and kill the tumor cells *in vitro.* However, the inherent defects of the arginine deiminase of *Pseudomonas pudita* (e.g., activity of the enzyme is low in the condition of a neutral pH environment and it can be eliminated quickly) limit its application in the treatment of tumors. It has been confirmed that another arginine deiminase from the *Mycoplasma arginini* with a molecular weight of 46,000 Da is able to keep active under a neutral pH condition and inhibit the growth of the tumor in experimental animal models (Takaku et al. Int. J. Cancer, 51:244-249 (1992) and USP 5,474,928). But the arginine deiminase, as a heterologous protein from the microbe, also has the problems of high antigenicity, short circulating half life, and easy degradation in the body of the experimental animal. Some reports show the protein coupled with polyethylene glycol can prolong the half life significantly, reduce the antigenicity treat tumors (C. M. Ensor et al. Cancer Research, 62: 5443-5550 (2002)). Although coupling with polyethtlene glycol can make the arginine deiminase be used in clnical treartment of tumors (P. A. Ascierto et al. J. Clin. Oncol., 22: 1815-1822 and 23: 7660-7668 (2005)), the used coupling mode is a multiple-site and inhomogeneous modification, which leads to non-uniform shapes of modified proteins and uncontrolled preparation quality. As a result, the potency and the drug metabolism of the modified protein products of different batches are hard to evaluate, and meanwhile, the difference of clinical treatment effects of patients are hard to explain, resulting from the inherent nonuniformity of the drug, which may severely affect the formulation of the pertinent treatment protocols for different tumor paients.

Compared with chemical drugs, the polypeptide and protein drugs have the advantages of low toxicity/side effects and little drug resistance etc. In order to achieve higher activity, bioavailability, and lower degradation *in vivo,* the protein drugs are usually administrated intravenousely. But in this context, the half-life of low molecular weight protein drugs would be very short, not only because of the degradation, but also the quick elimination via kidney. In blood, if the proteins' hydraulic radiuses are larger than that of albumin or their molecular weights are larger than 66kDa, they can be reserved stably in circulation. However, protein of lower molecular weight will be quickly eliminated from the blood via glomeruli. As such, in order to maintain the effective treatment concentration of low molecular weight proteins in blood, frequent intravenous administration is required. Although treatment in such a way could achieve the therapeutic effects, but it also causes inconveniences and pains to the patients and increases the cost of treatment. Meanwhile, long term administration of some drugs may cause some side effects, for instance, immunological reactions.

Arginine deiminase as a protein drug also has the disadvantages of short half life and high elimination rate in vivo. Moreover, because the arginine deiminase is from the pathogenic microorganism, it has a higher antigenicity, which may induce a strong immune response in a human body.

Using macromolecule polymers to modify proteins is a common method to change and control the dynamics characteristics of drugs, such as half-life, biological characteristics, and toxicological characteristic. The macromolecule polymer used to modify proteins should have, among others, good water-solubility, good biocompatibility, and little immunogenicity. The polyethylene glycol is a prevailing protein-modifying molecule. The polyethylene glycol has the amphipathic properties, which can be dissolved not only in water, but also in most of the organic solvents. Meanwhile, the polyethylene glycol is non-toxic, non-immunogenic, and highly soluble in water, and thus has been approved as a macromolecule polymer for drug preparation by many countries' drug administrations including SFDA of China as well as FDA of the U.S.A. Coupling the protein with macromolecule polymers, for example, the polyethylene glycol, can increase the protein's *in vivo* stability, decrease nonspecific adsorption and antigenicity. Once the conjugate reaches a certain molecular weight, the eliminating rate by kidney can be reduced effectively, which is an effective measure to prolong the half-life of the protein drug *in vivo* (Frokjaer S. et al. Nat. Rev. Drug Discov. 2005 Apr 4(4): 298-306; Harris JM. et al. Nat. Rev. Drug Discov. 2003 Mar 2(3): 214-21). The amino groups initially used as the reaction site in the modification of the polyethylene glycol were mainly α-amino group of N-terminus of the protein, and ε-amino group of the side chain of the lysine residue. The product of the reaction is a protein molecule non-specifically coupled with one or multiple PEG molecules. The modification of ε-amino group in the side chain of the lysine residue may generate modified isomerides due to the non-specific reaction sites.

Recently, aiming at the difference of isoelectric points between α-amino group of N-terminus of the protein and ε-amino group of the side chain of the lysine residue, polyethylene glycol modifying agents have been developed which specifically modify N-terminus of the protein and as a result, it is possible to obtain uniform modification products with modification at the identical site. Another protein site for modification with the polyethylene glycol is the mercapto group of the cysteine residue. Generally, the number of the mercapto groups is fewer than that of the amino groups in a protein, and thus modification of the mercapto groups is more specific. Using genetic engineering techniques, now it is possible to introduce a cysteine at any position of a protein to serve as a specific modification site. But introducing a cysteine as the modification site also has certain limitations because, for those proteins or polypeptides that do not contain cysteine residues, this may cause the crosslinking between the molecules, resulting in the loss of activity, and for those proteins that already contain cysteine residues, this may cause mispairing of disulfide bonds, resulting that such proteins cannot renature. In addition, the carboxyl group of the protein is also frequently used as a site for modification (Veronese FM et al. Drug Discov. Today. 2005 Nov 1;10(21):1451-8.). Modification techniques with polyethylene glycol have been successfully used in a plurality of protein drugs, including PEG-asparaginase (Graham ML Adv. Drug Deliv. Rev. 55, 1293-1302 Avramis Vassilios I. et al. WO9939732 and US6689762), PEG-adenosine deaminase (Levy Y et al. J. Pediatr. 113, 312-317; Davis S et al. ClinExp. Immunol. 46: 649-652; Hershfield MS et al. N Engl J Med 316 : 589-596), PEG-interferons such as PEG-interferon α2a and PEG-interferon α2b etc. (Bailon P et al. C. Bioconjug. Chem. 12, 195-202, Wang YS et al. Adv. Drug Deliv. Rev. 54, 547-570, Meng Xiantai et al. WO2005077421, Van Vlasselaer Peter et al. WO2004076474, Ballon Pascal Sebastlan et al. US2004030101, Karasiewicz Robert et al. EP0593868) etc. WO2004/001048 discloses pegylation of arginase.

Other representative macromolecule modifying agents include glucan, the polysucrose, starch, polyalanine, copolymer of adipic acid and the malonic acid, carboxymethyl cellulose, polyvinylpyrrolidone, poly 1,3-dioxolane, ethylene and maleic hydrazide copolymer, poly sialic acid, cyclodextrin, etc.

Another method of prolonging the half-life of a protein drug is to couple or fuse it with a blood protein or a fragment thereof used as a carrier to increase the molecular weight of the protein drug. For example, Fc fragment of immunoglobulin may be coupled with the target protein to prolong the half-life of the latter. For example, this strategy has been used in the Notch 1 receptor protein (Kitajewsky Jan et al. WO2005111072), erythropoietin (EPO) (Gillies Stephen D et al. WO2005063808), human somatropin (Kim Young Min et al. WO2005047337), etc. The plasma albumin is another commonly-used coupling carrier, which is used in the proteins such as some antibiotics, anti-inflammatory agents, and anti-oxidants (Ezrin Alan M et al. WO0117568, Otagiri Masaki et al. EP1571212), etc.

In addition to directly coupling the protein drug with some blood protein carriers *in vitro,* it is also possible to modify the drug protein *in vitro* to provide the drug protein with chemical reaction activity or high affinity for some molecules *in vivo,* so that the drug protein can react with some components of the blood upon entering into the body to form macromolecules or compounds with longer half-life. One example is the antiviral small peptide anti-RSV modified with an active group carrying maleimide, which can react with the mercapto groups of the blood proteins or the cell surface proteins (Bridon Dominique P et al. WO0069902). Another example is to introduce fatty acids to the amino acid residues on the surface of the protein by acylation reaction so as to increase the affinity of the protein to the albumin *in vivo.* Upon adminstered into the blood, the protein can form a larger conjugate with albumin and thereby the half-life of the protein is prolonged. The method has been used to produce long-acting insulins (Kurtzhals P et al. Biochem. J. (1995) 312, 725-731; Frokjaer S et al. Nat Rev Drug Discov. 2005 Apr;4(4):298-306).

Sustained release formulation is another approach of prolonging the *in vivo* half-life of a protein drug. The protein drug is placed in a pharmaceutical carrier, which may be a chemical macromolecule, or a physical device capable of releasing proteins slowly and continuously, to allow the protein to be released from the carrier slowly, and thereby a stable *in vivo* drug concentration is maintained. The commonly used sustained release formulations comprise hydrogel, microcapsule, microballoon, liposome, micro-osmotic pump, etc. (Peppas NA et al. Eur. J. Pharm. Biopharm. 50, 27-46 (2000); Packhaeuser CB et al. Eur. J. Pharm. Biopharm. 58, 445-455 (2004); Metselaar JM et al. Mini Rev. Med. Chem. 4, 319-329 (2002)). Liposome is an ultramicroscopic particle in the form of a hollow sphere with a bilayer membrane structure. The bilayer membrane is composed of amphipathic molecules, most of which are phosphatides, and forms a hydrophilic inner chamber. The hydrophilic protein drug is encapsulated in the inner chamber of the liposome and thus can be slowly released *in vivo* to maintain the concentration of the protein in blood and prolong the half-life. Examples are nerve growth factor (Hou Xinpu et al. CN1616087) and hemoglobin (Farmer Martha C et al. US4911929) etc. A micro osmotic pump is a physical device to control the content to be released slowly by using the difference between the exterior osmotic pressure and the interior osmotic pressure of the semi permeable membrane, which has been widely used in studying the slow-release of a variety of chemical and biological drugs in experimental animal models.

The main object of the present invention is to reduce the antigenicity of the arginine deiminase, to improve the metabolic characteristics of the protein in vivo, to make the protein be provided with higher stability and a longer half life in vivo, so that the protein can be used in clinical treatment of tumors, and the economic burden as well as the physiological pain of patients may be decreased.

### Summary of the Invention

The invention is defined by the following items:
1. A conjugate formed by coupling one polyethylene glycol molecule to one arginine deiminase molecule or a fragment thereof having arginine deiminase activity, wherein the polyethylene glycol molecule is coupled to the arginine deiminase or fragment thereof at the N-terminal a-amino group of the arginine deiminase or fragment thereof, and the polyethylene glycol has an average molecular weight in the range from 5,000 to 100,000 Daltons, preferably 5,000 to 40,000 Daltons, and more preferably 20,000 to 40,000 Daltons.
2. The conjugate of item 1, wherein said polyethylene glycol may be linear or branched.
3. The conjugate of item 1 or 2, wherein the polyethylene glycol has the molecular weight of 20,000 or 40,000 Daltons.
4. The conjugate of any one of items 1-3, wherein polyethylene glycol is monomethoxy polyethylene glycol or monohydroxyl polyethylene glycol.
5. The conjugate of any one of items 1-4, wherein the monomethoxy polyethylene glycol is monomethoxy polyethylene glycol butyrald (mPEG-ButyrALD) of 20,000 or 40,000 Daltons.
6. The conjugate of any one of items 1-5, wherein the arginine deiminase or fragment thereof is derived from *Mycoplasma hominis, Mycoplasma arthritidis,* or *Mycoplasma arginini,* or the arginine deiminase is an arginine deiminase of *Mycoplasma hominis, Mycoplasma arthritidis* or *Mycoplasma arginini* prepared by cloning via genetic recombination technology.
7. The conjugate of any one of items 1-6, wherein the arginine deiminase has a sequence as shown in SEQ ID No.1 or SEQ ID No.2.
8. The conjugate of any one of items 1-6, wherein the arginine deiminase has a sequence as shown in SEQ ID No.3 or SEQ ID No.4.
9. A sustained-release formulation formed by a conjugate of any one of items 1-8 with a bio-compatible carrier, wherein said sustained-release formulation is in a form selected from the group consisting of microcapsule, hydrogel, microsphere, micro-osmotic pump or liposome.
10. A pharmaceutical composition comprising the conjugate of any one of items 1-8 or the sustained-release formulation of item 9 and a pharmaceutically acceptable carrier.
11. The pharmaceutical composition of item 10, wherein the pharmaceutically acceptable carrier is selected from the group consisting of aqueous pH buffer solutions including buffer solutions of phosphate, citrate and other organic acids; antioxidants including ascorbic acid; polypeptides with a low molecular weight (no more than 10 residues); proteins such as serum albumin, glutin or immunoglobulin; hydrophilic polymers such as polyvinylpyrrolidone; amino acids such as glycine, glutamine, asparagine, arginine or lysine; monosaccharide, disaccharide and other carbohydrates including glucose, mannose or dextrin; chelating agents such as EDTA; sugar alcohols such as mannitol or sorbitol; salt-forming counter ions such as sodium ion; nonionic surfactants such as TWEEN®, PEG and PLURONICS®.
12. Use of the conjugate of any one of items 1-8, the sustained-release formulation of item 9, or the pharmaceutical composition of item 10 or 11 in the preparation of anti-tumor medicaments.
13. Use of the conjugate of any one of items 1-8, the sustained-release formulation of item 9, or the pharmaceutical composition of item 10 or 11 in the preparation of medicaments for preventing or treating a tumor.
14. A method for prolonging the in vivo half-life and anti-tumor efficacy of arginine deiminase, comprising forming a conjugate of any one of items 1-8, or forming a sustained-release formulation of item 9.

### Brief description of the drawings

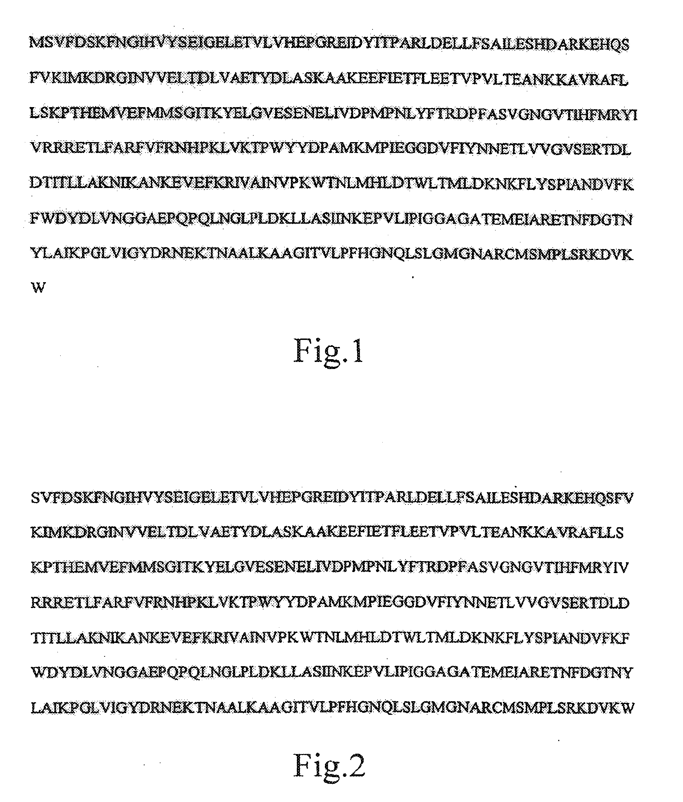

FIG. 1 depicts the amino acid sequence of a wild-type arginine deiminase from *Mycoplasma hominis* (SEQ ID No.1).
FIG. 2 depicts the amino acid sequence of a wild-type *Mycoplasma hominis* arginine deiminase expressed by *E. coli,* yeast or mammalian cells (SEQ ID No.2).

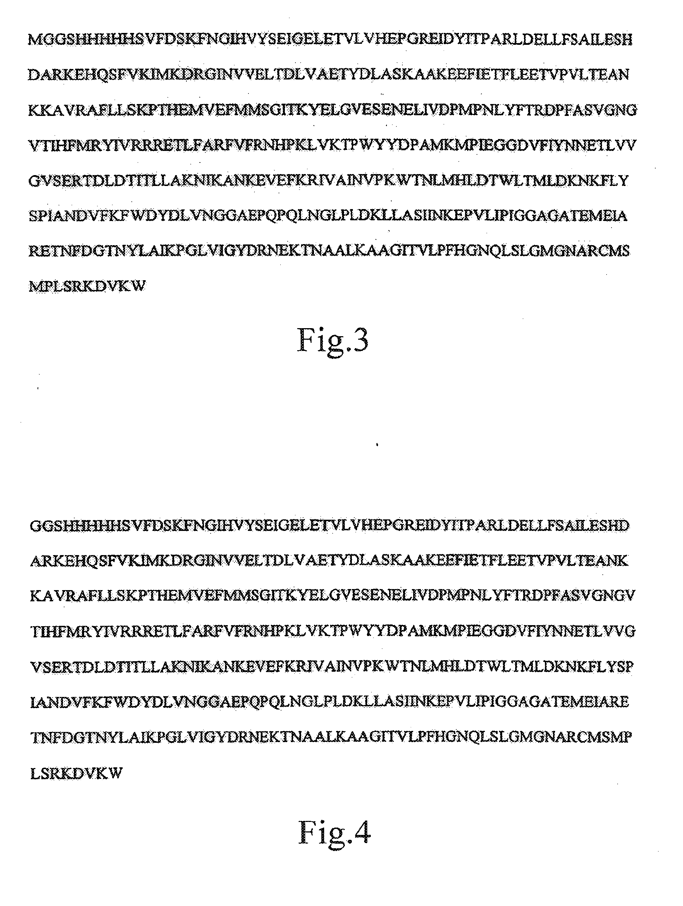

FIG. 3 depicts the amino acid sequence of *Mycoplasma hominis* arginine deiminase derivative expressed by *E. coli* (SEQ ID No.3).
FIG. 4 depicts the amino acid sequence of *Mycoplasma hominis* arginine deiminase derivative expressed by *E. coli,* yeast or mammalian cells (SEQ ID No.4).

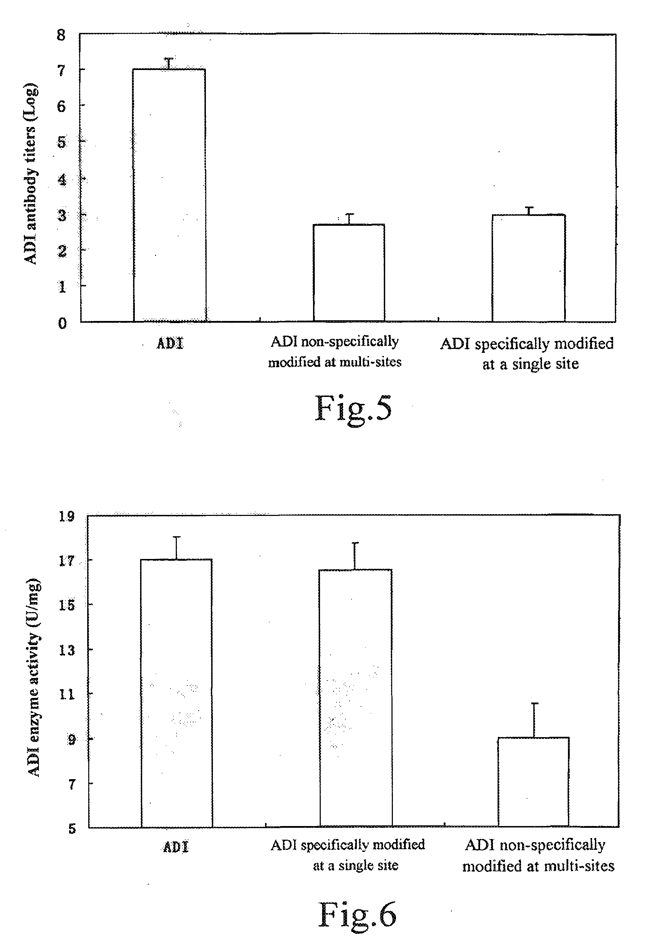

FIG. 5 is a graph showing the low antigenicity of Arginine Deiminase (ADI) coupled to polyethylene glycol at a single site of N-terminus in experimental animal. 9 healthy Kunming mice with an average body weight of around 25g were devided into 3 groups, tail-vein injected with arginine deiminase, arginine deiminase specifically modified with 20 kDa polyethylene glycol at a single site of the N-terminus and arginine deiminase non-specifically modified with polyethylene glycol at multi-sites respectively; the dosage is set 15 mg/kg body weight.
FIG. 6 is a graph showing that the arginine deiminase specifically modified with polyethylene glycol at a single site of the N-terminus preserves its integral biochemical activity. Arginine deiminase was dissolved in 100 µl phosphate buffered saline (PBS) to a final concentration of 10 µg/ml, and then L-arginine as the substrate of the arginine deiminase was added to a final concentration of 10 µmol/L. The reaction mixture system was incubated in 37 water bath for 10 minutes, and then the mixture was taken out to measure the change of L-arginine concentration using an assay kit following the instruction of the manufacturer.

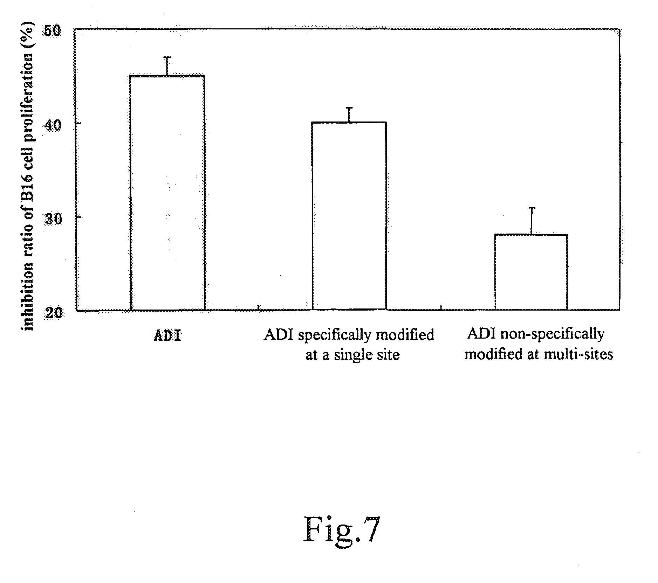

FIG. 7 is a graph showing that the arginine deiminase specifically modified with polyethylene glycol at a single site of the N-terminus preserves its intact activity of inhibiting tumor cell proliferation. The m ice malignant melanoma cells (B16/F10) were cultured in DMEM (10% serum added) until logarithmic phase. And then the cells were starved under serum-deprived condition for 12 hours. Normal culture media containing 10% fetal bovine serum and dual antibiotics were added to the tumor cells. Treatment groups were treated with arginine deiminase and modified arginine deiminase (including arginine deiminase specifically modified at a single site and arginine deiminase non-specifically modified at multi-sites) respectively to a final concentration of 10 µg/ml; and the control group is added with an equal volume of normal saline.

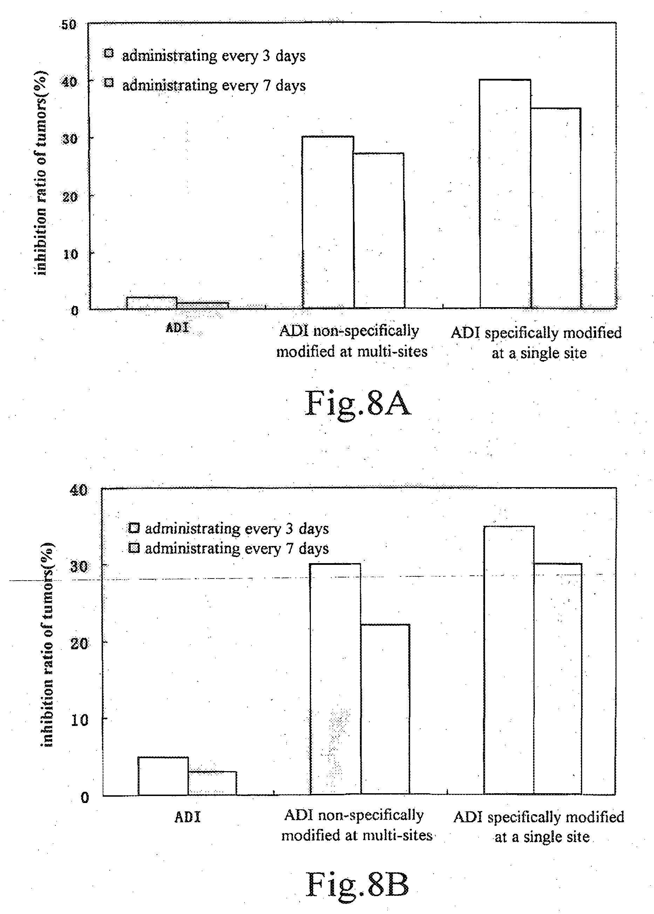

FIG. 8 depicts the activity of the arginine deiminase specifically modified with polyethylene glycol at a single site of the N-terminus in treating mouse tumor models. C57 mice with an average body weight of about 20g were given subaxillary inoculation with B16/F10 malignant melanoma cells, 2×10⁶ cells per mouse. The mice were randomly grouped the next day, 8 mice per group. Designated were the negative control group (normal saline), the positive control group (arginine deiminase 5mg/kg body weight (1.7U/mouse), daily administration), and the treatment groups were employed to treatment of arginine deiminase specifically modified at a single site and arginine deiminase non-specifically modified at multi-sites, with an administration interval of once every 3 days and once every 7 days, respectively. A, tail-vein administration group; B, subcutaneous administration group.

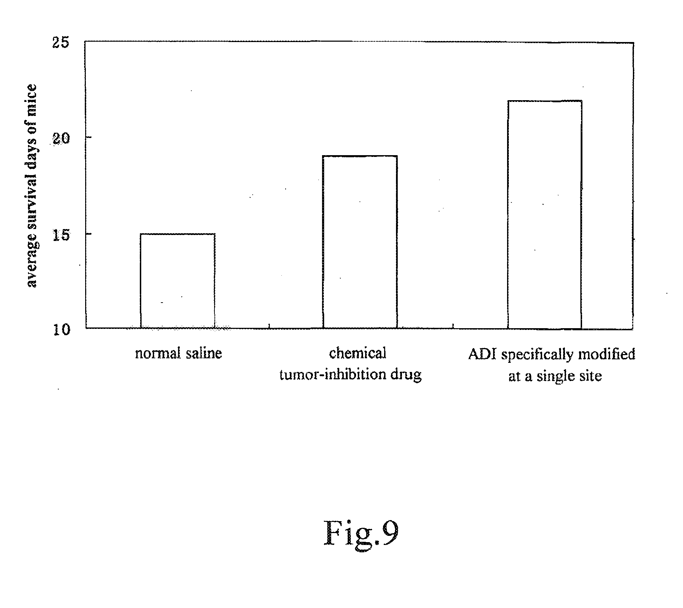

FIG. 9 is a graph showing that the arginine deiminase specifically modified with polyethylene glycol at a single site of the N-terminus significantly prolonged the survival time of tumor-bearing mice. C57 mice with an average body weight of about 20 g were given subaxillary inoculation with B16/F10 malignant melanoma cells, 2×10⁶ cells each mouse. The mice were randomly grouped the next day, 8 mice per group. Designated were the negative control (normal saline), the positive control (chemical tumor-inhibition drug, daily administration), the treatment group using arginine deiminase specifically modified at a single site (the interval of treatment is set once every 7 days), respectively.

### Detailed description of the invention

The arginine deiminase of the disclosure can be derived from any microorganisms possessing arginine deiminase, unless otherwise indicated. Preferably, the arginine deiminase is wild type *Mycoplasma homin is,* as shown in SEQ ID No.1. The arginine deiminase of the disclosure include wild type arginine deiminase (i.e., naturally existing form) or the mutant, fragment, isomer or derivative thereof having activity or their combinations. The arginine deiminase can be fermented and purified from *E.coli* (as shown in SEQ ID No.1 and SEQ ID No. 2), expressed in animal cells or fermented from yeast (as shown in SEQ ID No.2). Among them, the amino acid sequence of wild type *Mycoplasma hominis* arginine deiminase expressed in animal cells is shown in SEQ ID No.2; the amino acid sequence of recombinant Mycoplasma hominis arginine deiminase expressed in Yeast has the sequence as shown in SEQ ID No.2 or has a insertion or deletion of less than 10 amino acids at the N terminus of this sequence; and the recombinant Mycoplasma hominis expressed in *E.coli* has the sequence as shown in SEQ ID No. 1 or/and SEQ ID No. 2.

The invention provides a kind of arginine deiminase product with low antigenicity and a long half life. Compared with the unmodified arginine deiminase, it not only keeps its activity in inhibiting tumor cell growth, but also has very low antigenicity, higher stability in vivo and longer half life in vivo.

Polyethylene glycol or "PEG" refers to a mixture of branced or linear condensation polymers of ethylene oxide and water, represented by the general formula H(OCH₂CH₂)ₙOH wherein n is at least 4. "Polyethylene glycol" or "PEG" is used in combination with a numeric suffix to indicate the approximate average molecular weight thereof. For example, PEG 20,000 refers to polyethylene glycol having an average molecular weight of about 20,000 Daltons; PEG 40,000 refers to polyethylene glycol having an average molecular weight of about 40,000 Daltons.

This invention is based on a suprising discovery: arginine deiminase which is specifically modified with PEG at a single site also has significant effects in treating certain tumors and preventing tumor metastasis. Compared with multi-site modified arginine deiminase and arginine deiminase modified non-specifically at a single site, the arginine deiminase modified specifically at a single site not only preserves its low antigenicity, good tumor inhibiting activity but also possesses higher activity, better homogeneity, and the reproducity of the product quality of different batches remarkedly superior to non-specifically modified arginine deiminase, and thus can be utilized in clinical tumor treatment and the preparation of anti-tumor medicaments.

In the present invention, the gene encoding arginine deiminase may be obtained, cloned or produced from any source, for example, microorganisms, recombinant biotechnology or any combination thereof. Preferably, arginine deiminase is cloned from microorganisms of *Mycoplasma.* More preferably, the arginine deiminase is cloned from *Mycoplasma arginini, Mycoplasma hominis,* Mycoplasma arthritides, or any combination thereof. In particular, the arginine deiminase used in the present disclosure may have one or more of the amino acid sequences as shown in SEQ ID No.1.

A mutant of arginine deiminase refers to a protein molecule obtained by the replacement, deletion, insertion of amino acids. A fragment of arginine deiminase refers to a sequence belonging to any smaller part of SEQ ID No.1. The sequence can be obtained via enzyme cleavage, expressed by genentic engineering or obtained by polypeptide synthesis. An isomer of arginine deiminase refers to a molecule with the same amino acid sequence as the wild-type arginine deiminase but with different conformations, including difference in the secondary or tertiary protein structure, or different optical activity in regional amino acids. The isomer may be a naturally occurring mutant or obtained via artificial design. A derivat ive of arginine deiminase refers to a product generated by modification of the wild-type arginine deiminase. Modification refers to covalently attaching one or more small molecules, such as phosphoric acid molecules or carbohydrate molecules, or oligopeptide with less than 20 amino acids, to the protein at any amino acid of the protein. In a preferred embodiment, the derivative may be an arginine deiminase modified at N-termina with an addition of peptide containing a His-tag which may be consisted of 3 to 10 amino acids. The derivative preferably has the sequence shown in SEQ ID No.3 or SEQ ID No.4 and includes derivatives sequences on the sequence shown in SEQ ID No.3 or SEQ ID No.4. The combination of the active mutants, fragments, isomers or derivatives of arginine deiminase means that the product simultaneously has two or more of the modifications mentioned above, for example, but not limited to, a mutant of a fragment or a modified mutant, etc.

The invention provides a kind of arginine deiminase product having a long er half life. This product is consisted of arginine deiminase and a non-arginine deiminase modifying component. The arginine deiminase is defined as above, and the modifying component can be any form, including, but not limited to macromolecule polymers, protein molecules, peptides or chemicals in any other forms. Arginine deiminase and the modifying component are linked by either covalent bonds or non-covalent interaction to form a stable conjugate or a stable composition. This product has the biological activity of arginine deiminase and has a longer half life in vivo and much lower antigenicity than the unmodified arginine deiminase. Therefore, it can serve as an anti-tumor drug.

In one embodiment of the disclosure, it provides a conjugate of a modifying agent and arginine deiminase, namely, a modified arginine deiminase. The term "conjugate" used herein refers to a modified arginine deiminase. Modification refers to linking one or more modifying agents by covalent bonds directly or indirectly to arginine deiminase. The modifying agent can be a polymer or a protein or a fragment thereof that is biocompatible and can increase the half life of arginine deiminase in blood. The modifying agent can be either chemically coupled to arginine deiminase or linked to the arginine deiminase via fusion expression.

The macromolecule polymers as mentioned above refer to non-peptide macromolecule polymers, which can either have its own bioactivity or not. The suitable polymers include, but not limited to, polyenol compounds, polyether compounds, polyvinylpyrrolidone, poly amino acids, copolymer of divinyl ether and maleic anhydride, N-(2-hydroxypropyl)-methacrylamide, polysaccharide, polyoxyethylated polyol, heparin or its fragment, poly-alkyl-ethylene glycol and its derivatives, copolymers of poly-alkyl-ethylene glycol and its derivatives, poly(vinyl ethyl ether), a,P-Poly[(2-hydroxyethyl)-DL-aspartamide], polycarboxylates, poly oxyethylene-oxymethylenes, polyacryloyl morpholines, copolymer of amino compounds and oxyolefin, poly hyaluronic acid, polyoxiranes, copolymer of ethanedioic acid and malonic acid, poly (1,3-dioxolane), ethylene and maleic hydrazide copolymer, poly sialic acid, cyclodextrin, etc. Preferably, the polymer is polyethylene glycol.

The polyenol compound s as mentioned above include, but not limited to, polyethylene glycol (including monomethoxy polyethylene glycol, monohydroxyl polyethylene glycol), polyvinyl alcohol, polyallyl alcohol, polybutenol and the like, and their derivatives, such as lipids.

The polyether compounds include, but not limited to poly alkylene glycol (HO((CH2)ₓO)ₙH), polypropylene glycol, polyoxyrehylene(HO((CH₂)₂)ₙH), polyvinyl alcohol((CH₂CHOH)ₙ).

The poly amino acids as mentioned above include, but not limited to, polymers of one type of amino acid, copolymers of two or more types of amino acids, for example, polyalanine.

The polysaccharides as mentioned above include, but not limited to, glucosan and its derivatives, for example dextran sulfate, cellulose and its derivatives (including methyl cellulose and carboxymethyl cellulose), starch and its derivatives, polysucrose, etc.

In one specific embodiment of the present disclosure, arginine deiminase is coupled with proteins or peptides, wherein one or more proteins or peptides are directly or indirectly linked to arginine deiminase. The proteins can either be naturally existing proteins or their fragments, preferably but not limited to naturally existing human serum proteins or their fragments, including but not limited to thyroxine-binding protein, transthyretin, a1-acid glycoprotein, transferrin, fibrinogen, immunoglobulin, albumin and their fragments. 'Fragments' of a protein refer to any part of the protein that is smaller than the whole protein but retains the function of the protein as a carrier. Arginine deiminase is directly or indirectly linked to the carrier protein via a covalent bond. Direct linking means that one amino acid of arginine deiminase is directly linked to one amino acid of the carrier protein, via peptide bond or a disulfide bridge. Indirect linking refers to the linkages between arginine deiminase and carrier proteins, via originally existing chemical groups therebetween or specific chemical groups added through biological or chemical ways, or the combination of the abovementioned linkages.

In one embodiment of the present disclosure, the polyethylene glycol (PEG)-modified arginine deiminase is characterized in that one arginine deiminase molecule is covalently coupled to one PEG molecule. The coupling site of arginine deiminase may be one of the N-terminal α-amino group, the ε-amino group on the side chain of a lysine residue, the mercapto group of a cysteine residue, the carboxyl group on the side chain of an aspartate residue, the carboxyl group on the side chain of a glutamate residue, preferably but not limited to the N-terminal α-amino group. All the polyethylene glycol molucules used for coupling may be a linear or branched molecule, with a molecular weight of 1,000 to 100,000 Daltons , preferably but not limited to 5,000 to 40,000 Daltons, more preferably but not limited to 20,000 to 40,000 Daltons. It is preferred to use the N-terminal α-amino group of a PEG specifically modified arginine deiminase to form a product modified at a single site.

In one embodiment of the present disclosure, it involves the chemically reactive modified arginine deiminase which have chemically reactive groups and the ability to react with certain substances in blood and form a stable covalent bond in vivo. Such substances include, without limitation, molecules with one reactive group which can react and form a covalent bond with an amino group, a hydroxyl group, a mercapto and the like in blood. The active group is prefererally but not limited to maleimide which can react with the mercapto of blood proteins including but not limited to movable blood proteins e.g. albumin.

In one specific embodiment of the present disclosure, the conjugate comprising arginine deiminase is a conjugate molecule with a specific composition, which is formed via non-covalent interaction between arginine deiminase and other molecules. This conjugate has the activity to inhibit tumor growth and has longer *in vivo* half-life than arginine deiminase.

Another embodiment of the present disclosure relates to a sustained-release composition comprising arginine deiminase. The sustained-release composition is a stable composition consisting of arginine deiminase or the conjugate thereof and a pharmaceutical carrier. Arginine deiminase in this composition still has biological activity, and at the same time, its *in vivo* half-life is prolonged because of the carrier which changes the pharmacokinetic characteristics of the drug. It is preferred without limitation that the chemical or physical substained-release substances are used in the in vivo sustained-release technology. In a preferred embodiment, arginine deiminase or the conjugate, the composition thereof is embedded in a liposome. In another preferred embodiment, arginine deiminase or the conjugate, the composition thereof is contained in a micro-osmotic pump.

The present disclosure also provides a pharmaceutical composition which contains the arginine deiminase or its conjugate or composition. The pharmaceutical composition is composed of arginine deiminase-containing pharmaceutical composition and a proper pharmaceutical carrier. The pharmaceutical carriers used in this disclosure include carriers, excipients, or stabilizers, which are nontoxic to the cells or mammals to be contacted at selected doses and concentrations. A commonly used physiologically acceptable carrier is an aqueous pH buffer solution. Examples of physiologically acceptable carriers include solutions such as phosphate buffer solution, citrate buffer solution and other organic acid buffer solution; anti-oxidants including ascorbic acid; polypeptides with low molecular weight (no more than 10 residues); proteins such as serum albumin, glutin or immunoglobulin; hydrophilic polymers such as polyvinylpyrrolidone; amino acids such as glycine, glutamine, asparagine, arginine or lysine; monosaccharide, disaccharide and other carbohydrates including glucose, mannose or dextrin; chelating agents such as EDTA; sugar alcohols such as mannitol or sorbitol; salt-forming counter ions such as sodium ion; and/or nonionic surfactants such as TWEEN^{®}, PEG and PLURONICS^{®}.

The aforementioned pharmaceutical composition of arginine deiminase or its conjugate generally can be combined with pharmaceutical carriers (excipients) to form a pharmaceutical composition. The pharmaceutical carriers can comprise one or more physiologically acceptable compounds which can be used to stabilize such composition, or increase or decrease the absorption of active reagents. Physiologically acceptable compounds include, for example, carbohydrates such as glucose, sucrose or dextran; antioxidants such as antiscorbic acid or glutathione, chelating agents, proteins with a low molecular weight, protecting and absorption enhancers such as lipids, components which can decrease the elimination or hydrolysis of active reagents, excipients or other stabilizers and/or buffers.

Other physiologically acceptable compounds include wetting agents, emulsifying agents, dispersing agents, or protecting agents which are especially useful in preventing the growth or influences of micro organisms. Various protecting agents are well-known including phenol and antiscorbic acid. A person skilled in the art is able to expect that the selection of pharmaceutical carriers, which comprise physiologically acceptable compounds, depends on, e.g. the administration route and specific physiological-chemical characteristics of the active reagents

The preferred excipients are sterilized and usually free of dys-materials. These compounds can be sterilized through routine and well-known sterilizing techniques.

The present disclosure also provides a kit which includes the aforementioned arginine deiminase or its conjugate, composition and the operating instruction.

The present disclosure also involves methods for preparing the aforementioned conjugate or composition of arginine deiminase. In particular, the present disclosure relates to methods for preparing a conjugate of arginine deiminase which is coupled by PEG at a single site of N-terminus.

The present disclosure also provides methods of the prophylaxis, diagnosis or treatment of cancers and other arginine-related diseases as well as the inhibition of tumor growth and metastasis, wherein the the conjugates or compositions of arginine deiminase with low antigenicity, high activity and long half life are included without limitation as drugs. The tumors that are suitable to be treated with the method include, but not limited to, lung cancer, hepatoma, gastric cancer, esophageal cancer, bone cancer, pancreatic cancer, lymphoma, colon cancer, breast cancer, prostate ca ncer, oral cancer, nasopharyngeal carcinoma, uterine cervix cancer, leukemia, malignant melanoma, sarcoma, renal cancer, biliary cancer, etc. It is preferred without limitation that a conjugate of arginine deiminase which is coupled with PEG at a single site of N-terminus is used to treat tumors.

The present disclosure also provides the administration routes of the aforementioned conjugate or composition of arginine deiminase or both in the prophylaxis, diagnosis or treatment of tumors and other arginine-related diseases. The administration routes include, but not limited to, intravenous injection, intravenous drip, venous canal administration, arterial canal administration, intramuscular injection, intraperitoneal injection, oral administration, inhalation administration, subcutaneous administration, dermal administration, rectal administration, vaginal administration, nasal mucosa administration, oral mucosa administration, ocular administration, or other administration routes.

The present disclosure also provides a method and use of the abovementioned conjugate or composition of compositon of arginine deiminase or both in the preparation of anti-tumor medicaments.

PEG is directedly conjugated to the N-terminal amino acid residues of arginine deiminase, and the single modified protein product is purified. The single-site specifically modified arginine deiminase has a similar low antigenicity as multiple-site non-specifically modified arginine deiminase, which hardly induces immune responses in experimental animals; meanwhile, the single-site modified arginine deiminase has a higher activity than the multiple-site modified arginine deiminase at the same molar concentration; the single-site specifically modified arginine deiminase exhibites a higher activity in inhibiting tumor activity than multiple-site modified arginine deiminase in parallel treatments of mice suffering from tumors; the single-site specifically modified arginine deiminase exhibits a much better homogenicity and unicity of materials than multiple-site modified arginine deiminase in HPLC; the method for preparing single-site specifically modified arginine deiminase has a higher utilization rate of modifying materials, that is to say, fewer macromolecule material s are capable of producing more modified products.

It has been demonstrated that the conjugate of arginine deiminase modified by PEG at a single site ofN-terminus has a higher activity in inhibiting tumor cell proliferation and mice tumor growth and a higher stab ility and enzyme activity than multiple-site modified arginine deiminase and single-site non-specifically modified arginine deiminase with the same dosage; moreover, the former has a better component homogenicity, purity, structural homogenicity and reproducibility of product quality of different batches. Additionally, the modified product of arginine deiminase modified by PEG at a single-site of N-terminus can effectively reduce antigenicity and slow down the metabolism of arginine deiminase and thus increase the half life of arginine deiminase in *in vivo* pharmacokinetic experiments. In a preferred embodiment, the N-terminal PEG single-site modified arginine deiminase conjugate can be used for tumor treatment and for the preparation of anti-tumor medicaments.

At the same time, compared with the PEG non-directedly conjugated arginine deiminase, or PEG directedly conjugated other types of proteins, the N-terminus directedly conjugated arginine deiminase has increased for several times in preparation efficiency, enzyme activity; the dosage of the directedly conjugated arginine deiminase is much less to achieve the same level of tumor inhibition; although each of the arginine deiminase is modified with one PEG molecule, compared with the arginine deiminase modified by a plurity of PEG molecules with the same molecular weight, the former has a comparable effect in decreasing antigenicity and increasing half life.

In Poly(ethylene glycol) (PEG) conjugated arginine deiminase: effects of PEG formulations on its pharmacological properties Journal of Controlled Release, 80: 259 -271(2002), F.W. Holtsberg and his colleagues introduced the method for modification of arginine deiminase with PEG. It is described that, under a standard reaction condition, the molar ratio of PEG reagent to arginine deiminase of 40:1 was used for all PEGs ≤20 kDa and a molar ratio of 50:1 was used for all PEGs ≥30 kDa, and the reaction efficiency was typically ≥95%. In our experiments, a molar ratio of PEG reagent to arginine deiminase of 2:1 was used, the reaction efficiency commonly reached to 70%, so the preparation efficiency has increased by 10 times or more. Compared with other single PEG modified proteins, such as Interferon-β-1b, Bioconjugate Chem. 2006, 17, 618-630, the reaction efficiency has increased by approximately 5 times.

In the same article, F.W. Holtsberg et al have mentioned that, under general reaction conditions, the enzyme activity of multiple PEG modified arginine deiminase decreased by 40% to 65%, while the single PEG modified arginine deiminase has the same level of enzymatic activity as before modification, which is twice that of the multiple PEG modified product.

In C.M.Ensor et al, Cancer Research, 62: 5443-5450 (2002), Mike A. Clark et al, WO9851784, and Mike A. Clark et al, WO2002044360, the dosage of multiple PEG modified arginine deiminase is 5 IU/mouse/week in mice tumor models, however, in our experiments, the dosage of 1.7 IU/mouse/week also achieved a prominent tumor inhibition. It can be seen that the dosage in our experiments had decreased to 1/3 of the previously disclosed dosage.

The single PEG modified arginine deiminase of the present invention has a comparable level of decrease in antigenicity to the multiple PEG modified arginine deiminase with the same molecular weight as reported in F.W. Holtsberg et al. Journal of Controlled Release, 80: 259 -271(2002), Mike A. Clark et al, WO9851784, and Ascierto et al, Journal Of Clinical Oncology, 23:7660-7668 (2005) ; and in the respect of prolonging half life, weekly use of the single PEG modified arginine deiminase also achieved prominent treatement efficacy.

Other single PEG modified proteins, such as Interferon-β-1b, Bioconjugate Chem. 2006, 17, 618- 630 have a half life increased about 10 times, and the single PEG modified arginine deiminase of the present invention has a half life increased by approximately 20 times.

### Examples

### Example 1: Coupling of PEG to the N-terminus of arginine deiminase

The recombinant arginine deiminase (Protgen Ltd.) was dialyzed into 10 mM phosphate buffered saline, pH 7.0. Protein concentration was determined by measuring absorbance at 280 nm using UV spectrophotometer (Agilent Technologies), and then was adjusted to 4 mg/ml. When coupling with 20 kDa or 40 kDa PEG, 40 mg of 20 kD PEG (mPEG-ButyrALD 20 kDa, Nektar) solid or 80 mg of 40 kD PEG (mPEG-ButyrALD 40 kDa, Nektar) solid was added to 10 ml protein solution (containing 40 mg protein), and the mixture was stirred at room temperature until PEG solid dissolved completely and the molar ratio of PEG and arginine deiminase was 2:1. CH₃BNNa (Sigma) was added as reductant to achieve a final concentration of 20 mM, and the pH value of the solution was adjusted to 7. After resting at room temperature for 10 hours, most of the arginine deiminase was modified with mono-PEGylation, and a small amount of arginine deiminase was modified at multi-sites. The solution can be purified directly through column chromatography after being diluted to reduce ionic strength, or stored at 4 °C for short-term storage after being diluted 10-fold.

### Example 2: Purification of arginine deiminase modified with PEG at a single site of N-terminus through anion-exchange column

Arginine deiminase modified with 20 kDa or 40 kDa PEG was purified through anion-exchange column chromatography (Bio-Rad Ltd.). The pH value of the mixed solution after reaction was adjusted to 7. Sample was loaded onto column pre-equilibrated in a equilibrium buffer containing 10 mM-Tris, pH 7.0. After loading the sample, the chromatography column was eluted with 3 column volume of equilibrium buffer, and then gradient elution was performed with buffer containing 10 mM Tris, 0-1 M NaCl, pH 7.0. The PEG which did not involve in reaction did not attach to the column but the peak thereof appeared during penetration and washing due to its minimal charge. The elution peaks appeared in the following order: multi-site modified arginine deiminase, mono-site specifically modified arginine deiminase, and unmodified arginine deiminase. Different fractions can be collected according to absorbance at 280 nm.

### Example 3: The significant increase of the half-life in blood of arginine deiminase modified with PEG at a single site of N-terminus

The half-life of arginine deiminase and PEG modified arginine in mice was measured respectively to evaluate the prolonged efficacy of the modification with PEG. 6 healthy Kunming mice (the average body weight is about 25g) (Vitalriver Experimental Animal Center) were divided into 2 groups and injected with arginine deiminase and 20 kD PEG modified arginine deiminase via tail vein, in a dose of 15 mg / kg body weight. And then, blood samples were collected from tail vein at 2, 10, 30 minutes, 1, 2, 4, 8, 16, 24,48, 72, 96, 120, 144 and 168 hours. Plasma was stored at minus 80 °C. After blood was taken, the concentration of arginine deiminase and PEG modified arginine deiminase was measured through sandwich ELISA, respectively. *In vivo* pharmacokinetic result shows that in vivo half-life of arginine deiminase increases from an average of 4 hours to 72 hours after being modified with 20 kD PEG.

### Example 4: Low antigenicity of arginine deiminase modified with PEG at a single site of N-terminus in experimental animals

Immune responses induced by arginine deiminase, arginine deiminase specifically modified with PEG at a single site and arginine deiminase non -specifically modified at multi-sites were measured to examine the decrease of antigenicity after the modification with PEG at a single site (Figure 5). 9 healthy Kunming mice (the average body weight is about 25g) (Vitalriver Experimental Animal Center) were divided into 3 groups and injected with arginine deiminase, arginine deiminase specifically modified with 20 kD PEG at a single site of N-terminus and arginine deiminase non-specifically modified at multi-sites via tail vein, in a dose of 15 mg / kg body weight. Blood samples were collected from tail vein at 1, 7, 14, 21 days. Plasma was stored at minus 80 °C. After blood was taken, antibody titers against arginine deiminase in the blood of mice of each group were measured respectively through sandwich ELISA. Immunological result shows that the antibody titer induced by arginine deiminase modified with 20 kD PEG at a single site of N-terminus is about 10,000 times lower than that induced by wild-type unmodified arginine deiminase, and is just 2-3 times higher than that induced by arginine deiminase non-specifically modified with PEG at multi-sites.

### Example 5: Maintenance of the integral biochemical activity of arginine deiminase modified with PEG at a single site of N-terminus

Arginine deiminase specifically modified with PEG at a single site was used to examine whether the enzyme activity decreased due to the modification (Figure 6). Arginine deiminase was added to 100 ml phosphate buffered saline (PBS) to achieve a final concentration of 10 µg/ml, and then L-arginine was added as substrate of arginine deiminase to achieve a final concentration of 10 µM. The mixed reaction system was incubated for 10 minutes in a 37 °C bath, and the concentration change of L-arginine was measured by Blood Urea Nitrogen (BUN) kit (G-Cell Biotechnologies, Inc.). The result shows that the enzyme activities of unmodified arginine deiminase and arginine deiminase specifically modified at a single site are both 17 U / mg, which means that specific modification with 20 kDa PEG at a single site has no significant effect on the enzyme activity. We also used arginine deiminase non-specifically modified with PEG at multi-sites as a control. The result shows that the enzyme activity of arginine deiminase modified at multi-sites is only 50-60 % of that of arginine deiminase modified with PEG at a single site of N-terminus under same molar concentration, which means that the enzyme activity of arginine deiminase non-specifically modified with PEG at multi-sites decreased significantly.

### Example 6: The activity of inhibition of tumor cell proliferation by arginine deiminase modified with PEG at a single site of N-terminus

The inhibitory effect of 20 kDa PEG modified arginine deiminase on mice B16 / F10 malignant melanoma cell proliferation was observed (Figure 7). Mice malignant melanoma cells (B16/F10, ATCC # CRL-6475 ^{™}, USA) were cultured to logarithmic growth phase in DMEM medium (Hyclone) containing 10 % of serum, then starved in DMEM free of serum for 12 hours. Normal medium containing 10 % fetal bovine serum and double antibiotics (10 µ g / ml of streptomycin and ampicillin respectively, Sigma) was added. For treatment groups, arginine deiminase and modified arginine deiminase (including arginine deiminase specifically modified at a single site and arginine deiminase non-specifically modified at multi-sites) were added to achieve a final concentration of 10 µ g / ml, while for control group, an equal volume of normal saline was added. After incubation at 37°C for 24 hours, MTT was added to wells of the cell culture plate to achieve a final concentration of 0.25 mg/ml, and then the cells were incubated in 37°C incubators (Thermo Electron Corporation) for 6 hours and dissolved by DMSO (Shanghai Sangon Biological Engineering Technology & Services Co., Ltd.) finally. The cell number was counted under microscope. Cells in three different fields on one plate were counted and then the inhibition rate was calculated. The result shows that inhibition rate of arginine deiminase on tumor proliferation is 40 %, while inhibitory effect of arginine deiminase specifically modified at a single site on cell proliferation does not change. However, the enzyme activity of arginine deiminase modified at multi-sites is only about 70 % of that of arginine deiminase modified at a single site, which means that the enzyme activity of PEG specifically modified arginine deiminase is maintained completely, and compared with deiminase modified at multi-sites, PEG specifically modified arginine deiminase can better inhibit tumor cell growth *in vitro.*

### Example 7: Activity of arginine deiminase modified with PEG at a single site of N-terminus in the treatment of mice tumor models

The *in vivo* inhibitory effect of 20 kDa PEG modified arginine deiminase on mice B16 / F10 malignant melanoma was observed (Figure 8A, B). Each C57 mice (Vitalriver Experimental Animal Center) with an average weight of about 20 g were injected with 2×10⁶ B16 / F10 malignant melanoma cells via armpit. The mice were randomly grouped the next day, 8 mice per group. Designated were negative control group (normal saline), positive control group (arginine deiminase 5mg/kg body weight (1.7U/mouse), daily administration), and treatment groups which are employed to treatment of arginine deiminase specifically modified at a single site and arginine deiminase non-specifically modified at multi-sites, with an administration interval of once every 3 days and once every 7 days, respectively. Tumor-inoculated mice were administered after being divided into groups randomly via tail vein injection (Figure 8A) and subcutaneous injection from back and neck (Figure 8B) for 14 days. Then the mice were sacrificed, and tumor weights were measured on day 15. Tumor inhibition rate was calculated as follows to evaluate antitumor efficacy: tumor inhibition rate = (tumor weight of negative control group - tumor weight of treatment group) / tumor weight of negative control group × 100%. The result shows that the tumor inhibition rates of treatment group via tail vein injection per 3 days and per 7 days are 40 % and 30 % respectively; the tumor inhibition rates of treatment group via subcutaneous injection every 3 days and every 7 days are 35 % and 30 % respectively. Comparison of the tumor inhibition activities of arginine deiminase specifically modified at a single site and non-specifically modified at multi-sites shows that the tumor inhibition rate of arginine deiminase specifically modified at a single site is 10 % higher than that of arginine deiminase non-specifically modified at multi-sites under the same experimental condition. The result shows that unmodified arginine deiminase has no significant activity of tumor inhibition; specifically modified arginine deiminase has better *in vivo* activity of tumor inhibition than arginine deiminase non-specifically modified at multi-sites; the antitumor efficacy of specifically modified arginine deiminase can be maintained under the extended administration interval.

### Example 8: The significant effect of arginine deiminase modified with PEG at a single site of N-terminus on the extension of survival time of mice with tumors

The treatment effect of arginine deiminase modified with 40 kD PEG at a single site on the survival time of C57 mice with B16 / F10 malignant melanoma was observed (Figure 9). Each C57 mice with an average weight of about 20 g were injected with 2×10⁶ B16 / F10 malignant melanoma cells via armpit. The mice were randomly grouped the next day, 8 mice per group. Designated were the negative control (normal saline), the positive control (chemical tumor-inhibition drug, daily administration), the treatment group using arginine deiminase specifically modified at a single site (the interval of treatment was set once every 7 days), respectively. After being divided randomly into groups, tumor-inoculated mice were administered via subcutaneous injection from back and neck when the tumors reached an average diameter of about 2 cm. Treatment was performed over a period of 16 days, during which mice of each experimental group died one after another. The treatment effect was evaluated based on the average survival time of mice of each group. The result shows that the average survival time of mice of the negative control group, the positive control group and the treatment group is 15, 19 and 22 days, respectively.

## Claims

1. A conjugate formed by coupling one polyethylene glycol molecule to one arginine deiminase molecule or a fragment thereof having arginine deiminase activity, wherein the polyethylene glycol molecule is coupled to the arginine deiminase or fragment thereof at the N-terminal α-amino group of the arginine deiminase or fragment thereof, and the polyethylene glycol has an average molecular weight in the range from 5,000 to 100,000 Daltons, preferably 5,000 to 40,000 Daltons, and more preferably 20,000 to 40,000 Daltons.

2. The conjugate of claim 1, wherein said polyethylene glycol may be linear or branched.

3. The conjugate of claim 1 or 2, wherein the polyethylene glycol has the molecular weight of 20,000 or 40,000 Daltons.

4. The conjugate of any one of claims 1-3, wherein polyethylene glycol is monomethoxy polyethylene glycol or monohydroxyl polyethylene glycol.

5. The conjugate of any one of claims 1-4, wherein the monomethoxy polyethylene glycol is monomethoxy polyethylene glycol butyrald (mPEG-ButyrALD) of 20,000 or 40,000 Daltons.

6. The conjugate of any one of claims 1-5, wherein the arginine deiminase or fragment thereof is derived from *Mycoplasma hominis, Mycoplasma arthritidis,* or *Mycoplasma arginini,* or the arginine deiminase is an arginine deiminase of *Mycoplasma hominis, Mycoplasma arthritidis* or *Mycoplasma arginini* prepared by cloning via genetic recombination technology.

7. The conjugate of any one of claims 1-6, wherein the arginine deiminase has a sequence as shown in SEQ ID No.1 or SEQ 1D No.2.

8. The conjugate of any one of claims 1-6, wherein the arginine deiminase has a sequence as shown in SEQ ID No.3 or SEQ ID No.4.

9. A sustained-release formulation formed by a conjugate of any one of claims 1-8 with a bio-compatible carrier, wherein said sustained-release formulation is in a form selected from the group consisting of microcapsule, hydrogel, microsphere, micro-osmotic pump or liposome.

10. A pharmaceutical composition comprising the conjugate of any one of claims 1-8 or the sustained-release formulation of claim 9 and a pharmaceutically acceptable carrier.

11. The pharmaceutical composition of claim 10, wherein the pharmaceutically acceptable carrier is selected from the group consisting of aqueous pH buffer solutions including buffer solutions of phosphate, citrate and other organic acids; antioxidants including ascorbic acid; polypeptides with a low molecular weight (no more than 10 residues); proteins such as serum albumin, glutin or immunoglobulin; hydrophilic polymers such as polyvinylpyrrolidone; amino acids such as glycine, glutamine, asparagine, arginine or lysine; monosaccharide, disaccharide and other carbohydrates including glucose, mannose or dextrin; chelating agents such as EDTA; sugar alcohols such as mannitol or sorbitol; salt-forming counter ions such as sodium ion; nonionic surfactants such as TWEEN^{®}, PEG and PLURONICS®.

12. Use of the conjugate of any one of claims 1-8, the sustained-release formulation of claim 9, or the pharmaceutical composition of claim 10 or 11 in the preparation of anti-tumor medicaments.

13. Use of the conjugate of any one of claims 1-8, the sustained-release formulation of claim 9, or the pharmaceutical composition of claim 10 or 11 in the preparation of medicaments for preventing or treating a tumor.

14. A method for prolonging the *in vivo* half-life and anti-tumor efficacy of arginine deiminase, comprising forming a conjugate of any one of claims 1-8, or forming a sustained-release formulation of claim 9.

## Patentansprüche

1. Konjugat, das durch Kuppeln eines Polyethylenglykolmoleküls an ein Arginindeiminasemolekül oder ein Fragment davon mit Arginindeiminasewirkung ausgebildet wird, wobei das Polyethylenglykolmolekül an der N-endständigen α-Aminogruppe der Arginindeiminase oder des Fragments davon an die Arginindeiminase oder das Fragment davon gekuppelt ist und das Polyethylenglykol ein durchschnittliches Molekulargewicht im Bereich von 5.000 bis 100.000 Dalton, vorzugsweise 5.000 bis 40.000 Dalton und besonders bevorzugt 20.000 bis 40.000 Dalton, aufweist.

2. Konjugat nach Anspruch 1, wobei das Polyethylenglykol linear oder verzweigt sein kann.

3. Konjugat nach Anspruch 1 oder 2, wobei das Polyethylenglykol das Molekulargewicht von 20.000 oder 40.000 Dalton aufweist.

4. Konjugat nach einem der Ansprüche 1-3, wobei das Polyethylenglykol Monomethoxy-Polyethylenglykol oder Monohydroxy-Polyethylenglykol ist.

5. Konjugat nach einem der Ansprüche 1-4, wobei das Monomethoxy-Polyethylenglykol Monomethoxy-Polyethylenglykolbutyrald (mPEG-ButyrALD) mit 20.000 oder 40.000 Dalton ist.

6. Konjugat nach einem der Ansprüche 1-5, wobei die Arginindeiminase oder das Fragment davon von *Mycoplasma hominis*, *Mycoplasma arthritidis* oder *Mycoplasma arginini* abgeleitet ist oder die Arginindeiminase eine Arginindeiminase von *Mycoplasma hominis*, *Mycoplasma arthritidis* oder *Mycoplasma arginini* ist, die durch Klonen mittels genetischer Rekombinationstechnik hergestellt wird.

7. Konjugat nach einem der Ansprüche 1-6, wobei die Arginindeiminase eine Sequenz wie in SEQ ID Nr. 1 oder SEQ ID Nr. 2 gezeigt aufweist.

8. Konjugat nach einem der Ansprüche 1-6, wobei die Arginindeiminase eine Sequenz wie in SEQ ID Nr. 3 oder SEQ ID Nr. 4 gezeigt aufweist.

9. Retard-Formulierung, die durch ein Konjugat nach einem der Ansprüche 1-8 mit einem biokompatiblen Träger ausgebildet wird, wobei die Retard-Formulierung in einer Form ausgewählt aus der Gruppe bestehend aus Mikrokapsel, Hydrogel, Mikrokügelchen, osmotische Mikropumpe oder Liposom vorliegt.

10. Pharmazeutische Zusammensetzung, die das Konjugat nach einem der Ansprüche 1-8 oder die Retard-Formulierung nach Anspruch 9 und einen pharmazeutisch akzeptablen Träger umfasst.

11. Pharmazeutische Zusammensetzung nach Anspruch 10, wobei der pharmazeutisch akzeptable Träger aus der Gruppe bestehend aus wässrigen pH-Pufferlösungen, darunter Pufferlösungen von Phosphat, Citrat und anderen organischen Säuren; Antioxidationsmitteln, darunter Ascorbinsäure; Polypeptiden mit einem niedrigen Molekulargewicht (nicht mehr als 10 Reste); Proteinen wie z. B. Serumalbumin, Glutin oder Immunglobulin; hydrophilen Polymeren wie z. B. Polyvinylpyrrolidon; Aminosäuren wie z. B. Glycin, Glutamin, Asparagin, Arginin oder Lysin; Monosaccharid, Disaccharid und anderen Kohlenhydraten, darunter Glucose, Mannose oder Dextrin; Chelatbildnern wie z. B. EDTA; Zuckeralkoholen wie z. B. Mannitol oder Sorbitol; salzbildenden Gegenionen wie z. B. Natriumion; nichtionischen oberflächenaktiven Mitteln wie z. B. TWEEN®, PEG und PLURONICS® ausgewählt ist.

12. Verwendung des Konjugats nach einem der Ansprüche 1-8, der Retard-Formulierung nach Anspruch 9 oder der pharmazeutischen Zusammensetzung nach Anspruch 10 oder 11 bei der Herstellung von Antitumormedikamenten.

13. Verwendung des Konjugats nach einem der Ansprüche 1-8, der Retard-Formulierung nach Anspruch 9 oder der pharmazeutischen Zusammensetzung nach Anspruch 10 oder 11 bei der Herstellung von Medikamenten zur Prävention oder Behandlung eines Tumors.

14. Verfahren zum Verlängern der *in-vivo*-Halbwertszeit und der Antitumorwirksamkeit von Arginindeiminase, welches das Ausbilden eines Konjugats nach einem der Ansprüche 1-8 oder Ausbilden einer Retard-Formulierung nach Anspruch 9 umfasst.

## Revendications

1. Conjugué formé par le couplage d'une molécule de polyéthylène-glycol à une molécule d'arginine déiminase ou un fragment de celle-ci ayant une activité d'arginine déiminase, dans lequel la molécule de polyéthylène-glycol est couplée à l'arginine déiminase ou un fragment de celle-ci au groupe α-amino N-terminal de l'arginine déiminase ou un fragment de celle-ci, et dans lequel le polyéthylène-glycol a un poids moléculaire moyen dans la plage de 5 000 à 100 000 daltons, de préférence 5 000 à 40 000 daltons, et plus préférentiellement de 20 000 à 40 000 daltons.

2. Conjugué selon la revendication 1, dans lequel ledit polyéthylène-glycol peut être linéaire ou ramifié.

3. Conjugué selon la revendication 1 ou 2, dans lequel le polyéthylène-glycol a un poids moléculaire de 20 000 ou de 40 000 daltons.

4. Conjugué selon l'une quelconque des revendications 1 à 3, dans lequel le polyéthylène-glycol est du monométhoxy polyéthylène-glycol ou du monohydroxy polyéthylène-glycol.

5. Conjugué selon l'une quelconque des revendications 1 à 4, dans lequel le monométhoxy polyéthylène-glycol est du butyrald monométhoxy polyéthylène-glycol (mPEG-ButyrALD) de 20 000 ou de 40 000 daltons.

6. Conjugué selon l'une quelconque des revendications 1 à 5, dans lequel l'arginine déiminase ou un fragment de celle-ci est dérivée de *Mycoplasma hominis, Mycoplasma arthritidis* ou *Mycoplasma arginini* ou l'arginine déiminase est une arginine déiminase de *Mycoplasma hominis, Mycoplasma arthritidis* ou *Mycoplasma arginini* préparée par clonage par une technologie de recombinaison génétique.

7. Conjugué selon l'une quelconque des revendications 1 à 6, dans lequel l'arginine déiminase a une séquence telle qu'illustrée dans SEQ ID N° 1 ou SEQ ID N° 2.

8. Conjugué selon l'une quelconque des revendications 1 à 6, dans lequel l'arginine déiminase a une séquence telle qu'illustrée dans SEQ ID N° 3 ou SEQ ID N° 4.

9. Formulation à libération prolongée formée par un conjugué selon l'une quelconque des revendications 1 à 8, avec un porteur bio-compatible, dans laquelle ladite formulation à libération prolongée se présente sous une forme choisie dans le groupe consistant en microcapsule, hydrogel, microsphère, micro-pompe osmotique ou liposome.

10. Composition pharmaceutique comprenant le conjugué de l'une quelconque des revendications 1 à 8 ou la formulation à libération prolongée de la revendication 9 et un porteur acceptable sur le plan pharmaceutique.

11. Composition pharmaceutique selon la revendication 10, dans laquelle le porteur acceptable sur le plan pharmaceutique est choisi dans le groupe consistant en des solutions tampon pH aqueuses incluant des solutions tampon de phosphate, citrate et autres acides organiques ; des antioxydants incluant l'acide ascorbique ; des polypeptides de faible poids moléculaire (10 résidus maximum) ; des protéines telles que sérum albumine, glutine ou immunoglobuline ; des polymères hydrophiles tels que polyvinylpyrrolidone ; des acides aminés tels que glycine, glutamine, asparagine, arginine ou lysine ; du monosaccharide, du disaccharide, et d'autres glucides incluant le glucose, le mannose ou la dextrine ; des agents chélatant tels que EDTA ; des alcools de sucre tels que mannitol ou sorbitol ; des contre-ions formant des sels, tels que ion sodium ; des agents tensio-actifs non ioniques, tels que TWEEN®, PEG et PLURONICS®.

12. Utilisation du conjugué selon l'une quelconque des revendications 1 à 8, de la formulation à libération prolongée selon la revendication 9 ou de la composition pharmaceutique selon la revendication 10 ou 11, dans la préparation de médicaments anti-tumoraux.

13. Utilisation du conjugué selon l'une quelconque des revendications 1 à 8, de la formulation à libération prolongée selon la revendication 9 ou de la composition pharmaceutique selon la revendication 10 ou 11, dans la préparation de médicaments pour prévenir ou traiter une tumeur.

14. Procédé pour prolonger la demi-vie *in vivo* et l'efficacité anti-tumorale de l'arginine déiminase, comprenant la formation d'un conjugué selon l'une quelconque des revendications 1 à 8 ou la formation d'une formulation à libération prolongée selon la revendication 9.
